(19) Europäisches Patentamt
European Patent Office
Office européen des brevets

(11) Veröffentlichungsnummer: **0 074 074**
**B1**

# (12) EUROPÄISCHE PATENTSCHRIFT

(45) Veröffentlichungstag der Patentschrift: **27.12.85**

(51) Int. Cl.⁴: **C 07 C 127/22**, A 01 N 47/34

(21) Anmeldenummer: **82108026.4**

(22) Anmeldetag: **01.09.82**

(54) **N-Benzoyl-N'-phenoxyphenylharnstoffe, Verfahren zu ihrer Herstellung und ihre Verwendung zur Bekämpfung von Insekten.**

(30) Priorität: **09.09.81 DE 3135635**

(43) Veröffentlichungstag der Anmeldung: **16.03.83 Patentblatt 83/11**

(45) Bekanntmachung des Hinweises auf die Patenterteilung: **27.12.85 Patentblatt 85/52**

(84) Benannte Vertragsstaaten: **BE CH DE FR GB IT LI NL**

(56) Entgegenhaltungen: **DE - A - 2 504 982**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Parg, Adolf, Dr., Paray-le-Monial-Strasse 8, D-6702 Bad Duerkheim (DE)**
Erfinder: **Lange, Arno, Dr., Friedrichsplatz 11, D-6800 Mannheim (DE)**
Erfinder: **Adolphi, Heinrich, Dr., Kalmitweg 11, D-6703 Limburgerhof (DE)**

**EP 0 074 074 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue N-Benzoyl-N'-phenoxyphenylharnstoffe, Verfahren zu ihrer Herstellung, insektizide Mittel, die diese Verbindungen als Wirkstoffe enthalten sowie Verfahren zur Bekämpfung von Insekten mit diesen Wirkstoffen.

Insektizid wirksame N-Benzoyl-N'-phenoxyphenylharnstoffe sind aus der DE-OS 25 31 202 und der DE-OS 25 04 982 bekannt. Diese Wirkstoffe tragen jedoch im Phenoxyteil stets einen Substituenten in para-Stellung zur Etherbrücke.

Außerdem ist bekannt, daß N-2,6-Dichlorbenzoyl-N'-4-chlorphenoxyphenylharnstoff nur schwache insektizide Eigenschaften hat (J. Agric. Food Chem. 21, S. 353 (1973)). Daher überrascht es, daß die bislang unbekannten meta-substituierten Phenoxyphenylharnstoffe starke insektizide Wirkung zeigen.

Es wurde gefunden, daß N-Benzoyl-N'-phenoxyphenylharnstoffe der Formel

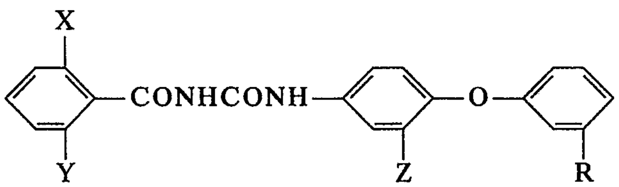

(I)

in der

X für Chlor, Fluor oder Methyl,
Y für Wasserstoff, Fluor oder Chlor,
Z für Wasserstoff, Fluor, Chlor, Brom, Alkyl mit 1 bis 8 Kohlenstoffatomen oder Trifluormethyl, und
R für Halogen, Alkyl mit 1 bis 8 Kohlenstoffatomen, Alkoxy, Halogenalkyl, Halogenalkoxy, Alkylmercapto und Halogenalkylmercapto mit 1 bis 4 Kohlenstoffatomen stehen, starke insektizide Eigenschaften besitzen. Sie sind bekannten N-Benzoyl-N'-phenoxyphenylharnstoffen in ihrer Wirkung überlegen.

Für Z und R in Formel I kommen unverzweigte oder verzweigte Akylreste mit 1 bis 8, vorzugsweise mit 1 bis 4 Kohlenstoffatomen, wie Methyl, Ethyl, n-Propyl, i-Propyl, n-Butyl, sec.-Butyl, tert.-Butyl, insbesondere Methyl, in Betracht.

R in Formel I steht außerdem für Halogen, insbesondere Fluor oder Chlor für unverzweigte oder verzweigte Alkoxy-, Halogenalkyl-, Halogenalkoxy-, Alkylmercapto- oder Halogenalkylmercaptoreste mit 1 bis 4 Kohlenstoffatomen, wie Methoxy, Ethoxy, n-Propoxy, i-Propoxy, n-Butoxy, Methylmercapto, Ethylmercapto, n-Butylmercapto, Trifluormethyl, 2,2,1,1-Tetrafluorethyl, Trifluormethoxy, 2-Chlordifluorethoxy, Chlordifluormethyl, Trifluormethylmercapto, 2,2,1,1-Tetrafluorethylmercapto, n-Propylmercapto.

Bevorzugte n-Benzoyl-N'-phenoxyphenylharnstoffe sind Verbindungen der Formel I, bei denen X Fluor oder Chlor, Y Fluor, Wasserstoff oder Chlor, Z Wasserstoff, Fluor oder Chlor und R Methyl, Fluor, Chlor oder Trifluormethyl bedeuten.

Man erhält die N-Benzoyl-N'-phenoxyphenylharnstoffe der Formel I durch Umsetzung von Benzoylisocyanaten der Formel

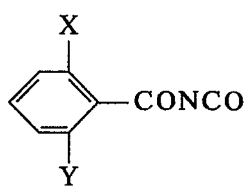

(II)

in der X und Y die obengenannten Bedeutungen haben,
mit phenoxysubstituierten Anilinen der Formel

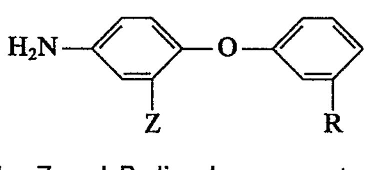

(III)

in der Z und R die obengenannten Bedeutungen haben, in Gegenwart eines inerten organischen Lösungsmittels bei einer Temperatur im Bereich zwischen 0 und 80°C. Die Umsetzung wird in Gegenwart geeigneter Lösungs- und Verdünnungsmittel durchgeführt. Als solche kommen praktisch alle inerten organischen Solventien in Betracht. Hierzu gehören insbesondere aliphatische und aromatische, gegebenenfalls chlorierte oder nitrierte Kohlenwasserstoffe, wie Benzol, Toluol, Xylole, Chlorbenzole, Benzin, Tetrachlorkohlenstoff, 1,2-Dichlorethan, Methylenchlorid, Chloroform, Nitromethan; cyclische und acyclische Ether, wie Diethylether, Dibutylether, Tetrahydrofuran, Dioxan; acyclische und cyclische

Ketone, wie Aceton, Methylethylketon, Methylisopropylketon, Cyclohexanon; Nitrile, wie Acetonitril, Benzonitril. Auch Gemische dieser Lösungsmittel können verwendet werden.

Die Reaktionstemperatur kann innerhalb eines größeren Bereichs variiert werden. Sie liegt in der Regel zwischen 0 und 80°C. Die Umsetzung verläuft aufgrund der exothermen Reaktion üblicherweise bei einer Temperatur im Bereich zwischen 20 und 60°C.

Man läßt die Umsetzung im allgemeinen bei Normaldruck ablaufen. Sie kann diskontinuierlich oder kontinuierlich durchgeführt werden.

Zur Durchführung der Verfahren setzt man die Reaktionskomponenten vorzugsweise in äquimolarem Verhältnis ein. Ein Überschuß der einen oder anderen Komponente bringt keine wesentlichen Vorteile. Die Umsetzung verläuft praktisch quantitativ.

Die Harnstoffe der Formel I fallen als Festprodukte an, die in der Regel analysenrein sind, andernfalls aber durch Umkristallisieren gereinigt werden können. Zu ihrer Charakterisierung dienen Elementaranalyse und Schmelzpunkt.

Zur Durchführung des erfindungsgemäßen Verfahrens wird zweckmäßigerweise das substituierte Phenoxyanilin der Formel III zusammen mit dem Lösungs- oder Verdünnungsmittel vorgelegt, dann wird das Isocyanat der Formel II zugegeben. Nach mehrstündiger Reaktionsdauer, in der Regel nach 2 Stunden, wird das Endprodukt dann abgesaugt und unter vermindertem Druck getrocknet.

Methoden der Herstellung für die Benzoylisocyanate der Formel II und der phenoxysubstituierten Aniline der Formel III sind bekannt (DE-OS 25 38 178; Ann. 740, 169 bis 179 (1970)).

Die Harnstoffe der Formel I können auch durch Umsetzung von Amiden der Formel

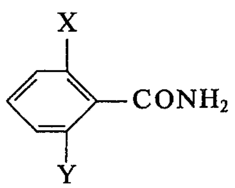

(IV)

mit Isocyanaten der Formel

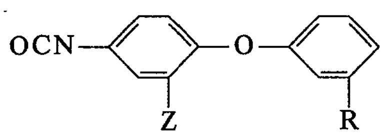

(V)

hergestellt werden. In den Formeln IV und V haben X, Y, Z und R die obengenannten Bedeutungen.

Die Reaktionspartner werden hierbei in äquimolaren Mengen gegebenenfalls in Gegenwart eines inerten organischen Lösungsmittels umgesetzt. Die Reaktionstemperatur liegt zwischen 0 und 140°C, vorzugsweise zwischen 60 und 100°C. Gegebenenfalls kann ein Katalysator, wie Triethylamin, zugesetzt werden. Als Lösungsmittel kommen die gleichen Solventien in Betracht, die bei der Umsetzung der Verbindungen der Formel II mit den Verbindungen der Formel III verwendet werden können.

## Herstellungsbeispiel

12,66 Gewichtsteile 4-3'-Trifluormethylphenoxy-anilin werden in 100 Volumteilen absolutem Acetonitril gelöst und tropfenweise mit 9,16 Gewichtsteilen 2,6-Difluorbenzoylisocyanat versetzt. Dann hält man für 2 Stunden bei 50°C und saugt bei 0°C ab. Anschließend trocknet man den Rückstand unter vermindertem Druck bei 40°C im Trockenschrank. Man erhält 19 Gewichtsteile N-(2,6-Difluorbenzoyl)-N'-[3'-trifluormethylphenoxy-phenyl]-harnstoff (Verbindung Nr. 1) vom Schmelzpunkt 189 bis 90°C.

Gewichtsteile verhalten sich zu Volumteilen wie kg zu Liter.

Folgende Verbindungen der Formel I lassen sich beispielsweise nach einem der oben beschriebenen Verfahren herstellen.

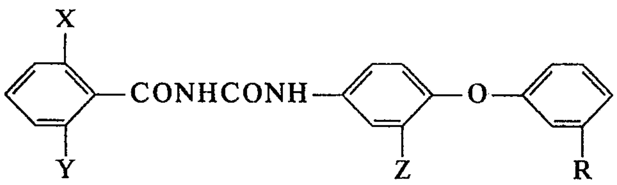

(I)

| Nr. | X | Y | Z | R | Fp. [°C] |
|---|---|---|---|---|---|
| 2 | Cl | H | H | Trifluormethyl | 166–168 |
| 3 | Cl | Cl | H | desgl. | 217–220 |
| 4 | $CH_3$ | H | H | desgl. | 173–175 |
| 5 | Cl | H | Trifluormethyl | desgl. | |
| 6 | F | F | desgl. | desgl. | 145–150 |
| 7 | Cl | H | Cl | desgl. | |
| 8 | Cl | Cl | Cl | desgl. | 157–160 |
| 9 | F | F | Cl | desgl. | 169–171 |
| 10 | Cl | H | F | desgl. | |
| 11 | Cl | Cl | F | desgl. | |
| 12 | F | F | F | desgl. | |
| 13 | Cl | H | H | Trifluormethoxy | |
| 14 | Cl | Cl | H | desgl. | |
| 15 | F | F | H | desgl. | 168–170 |
| 16 | Cl | H | H | Cl | 169–170 |
| 17 | Cl | Cl | H | Cl | 228–229 |
| 18 | F | F | H | Cl | 183–186 |
| 19 | Cl | H | H | Br | 182–184 |
| 20 | Cl | Cl | H | Br | 236–238 |
| 21 | F | F | H | Br | 202–204 |
| 22 | Cl | H | H | F | |
| 23 | Cl | Cl | H | F | |
| 24 | F | F | H | F | 179–182 |
| 25 | F | F | Cl | Cl | |
| 26 | F | F | Cl | Br | |
| 27 | F | F | Cl | F | |
| 28 | Cl | H | H | Methyl | 181–184 |
| 29 | Cl | Cl | H | desgl. | |

Fortsetzung

| Nr. | X | Y | Z | R | Fp. [°C] |
|---|---|---|---|---|---|
| 30 | F | F | H | Methyl | |
| 31 | F | F | H | Ethyl | |
| 32 | F | F | H | tert.-Butyl | 157–159 |
| 33 | F | F | H | Methoxy | 165–168 |
| 34 | Cl | H | H | Trifluormethylmercapto | |
| 35 | Cl | Cl | H | desgl. | |
| 36 | F | F | H | desgl. | |
| 37 | Cl | H | Cl | desgl. | |
| 38 | Cl | Cl | Cl | desgl. | |
| 39 | F | F | Cl | desgl. | |
| 40 | F | F | H | Methylmercapto | |
| 41 | $CH_3$ | H | H | Br | 183–185 |
| 42 | F | H | H | Cl | 150–152 |
| 43 | $CH_3$ | H | H | Cl | 173–175 |
| 44 | F | H | H | Br | 160–162 |
| 45 | F | Cl | H | $CF_3$ | |
| 46 | F | Cl | H | Cl | |
| 47 | F | Cl | | $CF_3$ | |
| 48 | Cl | Cl | H | Trifluormethylmercapto | |
| 49 | F | F | H | $OCF_2CF_2H$ | 158–161 |
| 50 | Cl | Cl | H | $OCF_2CF_2H$ | |
| 51 | Cl | H | H | $OCF_2CF_2H$ | |

Die N-Benzoyl-N'-phenoxyphenylharnstoffe der Formel I sind geeignet, Schädlinge aus der Klasse der Insekten und Spinnentiere wirksam zu bekämpfen. Sie sind geeignete Adultizide und Ovizide und können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyrethia conjugella (Apfelmotte), Sitotroga cercealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulan (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agricotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), atta sexdens (Blattschneiderameise);

aus der Ordnung Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Arachnoidea gehören Spinnentiere (Acarina), beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 3 Gewichtsteile der Verbindung Nr. 1 werden mit 97 Gewichtsteilen feinteiligem Kaolin innig vermischt. Man erhält auf diese Weise ein Stäubemittel, das 3 Gew.-% des Wirkstoffs enthält.

II. 30 Gewichtsteile der Verbindung Nr. 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 9 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 15 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 8 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mt gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix) zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan,
1,3-Dichlorpropen,
1,3-Dichlorpropen + 1,2-Dichlorpropan,
1,2-Dibrom-ethan,
2-sec.-Butylphenyl-N-methylcarbamat,
o-Chlorphenyl-N-methylcarbamat,
3-Isopropyl-5-methylphenyl-N-methylcarbamat,
o-Isopropoxyphenyl-N-methylcarbamat,
3,5-Dimethyl-4-methylmercapto-phenyl-N-methylcarbamat,
4-Dimethylamino-3,5-xylyl-N-methylcarbamat,
2-(1,3-Dioxolan-2-yl)-phenyl-N-methylcarbamat,
1-Naphthyl-N-methylcarbamat,
2,3-Dihydro-2,2-dimethyl-benzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)oxy]-1-thiooxamidat,
N-(2-Methyl-chlor-phenyl)-N',N'-dimethylformamidin,
Tetrachlorthiophen,
1-(2,6-Difluorbenzoyl)-3-(4-chlor-phenyl)-harnstoff,
O,O-Dimethyl-O-(p-nitrophenyl)-phosphorthioat,
O,O-Diethyl-O-(p-nitrophenyl)-phosphorthioat,
O-Ethyl-O-(p-nitrophenyl)-phenyl-phosphonothioat,
O,O-Dimethyl-O-(3-methyl-4-nitrophenyl)-phosphorthioat,
O,O-Diethyl-O-(2,4-dichlorphenyl-phosphorthioat,
O-Ethyl-O-(2,4-dichlorphenyl)-phenyl-phosphonothioat,
O,O-Dimethyl-O-(2,4,5-trichlorphenyl)-phosphorthioat,
O-Ethyl-O-(2,4,5-trichlorphenyl)-ethyl-phosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(3-methyl-4-methylthiophenyl)-phosphorthioat,
O-Ethyl-O-(3-methyl-4-methylthiophenyl)-isopropyl-phosphoramidat,
O,O-Diethyl-O-[p-methylsulfinyl-phenyl]-phosphorthioat,
O-Ethyl-S-phenyl-ethyl-phosphonodithioat,
O,O-Diethyl-[2-chlor-1-(2,4-dichlorphenyl)-vinyl]-phosphat,
O,O-Dimethyl-[2-chlor-1-(2,4,5-trichlorphenyl)]-vinyl-phosphat,
O,O-Dimethyl-S-(1'-phenyl)-ethylacetat-phosphordithioat,
Bis-(dimethylamino)-fluorphosphinoxid,
Octamethyl-pyrophosphoramid,
O,O,O,O-Tetraethyldithio-pyrophosphat,
S-Chlormethyl-O,O-diethyl-phosphordithioat,
O-Ethyl-S,S-dipropyl-phosphordithioat,
O,O-Dimethyl-O-2,2-dichlorvinyl-phosphat,
O,O-Dimethyl-1,2-dibrom-2,2-dichlorethylphosphat,
O,O-Dimethyl-2,2,2-trichlor-1-hydroxy-ethylphosphonat,
O,O-Dimethyl-S-[1,2-biscarbethoxy-ethyl-(1)]-phosphordithioat,
O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioat,
O,O-Dimethyl-S-(N-methoxyethyl-carbamoyl-methyl)-phosphordithioat,
O,O-Dimethyl-S-(N-formyl-N-methyl-carbamoylmethyl-phosphordithioat,

O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diethylcarbamoyl)-vinyl]-phosphat,
O,O-Diethyl-S-(ethyl-thio-methyl)-phosphordithioat,
O,O-Diethyl-S-[(p-chlor-phenylthio)-methyl]-phosphordithioat,
O,O-Dimethyl-S-(2-ethylthioethyl)-phosphorthioat,
O,O-Dimethyl-S-(2-ethylthioethyl)-phosphordithioat,
O,O-Dimethyl-S-(2-ethylsulfinyl-ethyl)-phosphorthioat,
O,O-Diethyl-S-(2-ethylthio-ethyl)-phosphordithioat,
O,O-Diethyl-S-(2-ethylsulfinyl-ethyl)-phosphorthioat,
O,O-Diethyl-thiophosphoryliminophenyl-acetonitril,
O,O-Diethyl-S-(2-chlor-1-phthalimidoethyl)-phosphordithioat,
O,O-Diethyl-S-[6-chlor-benzoxazolon-(2)-yl(3)]-methyldithiophosphat,
O,O-Dimethyl-S-[2-methoxy-1,3,4-thiadiazol-5-[4H]-onyl-(4)-methyl]-phosphordithioat,
O,O-Diethyl-O-[3,5,6-trichlor-pyridyl-(2)]-phosphorthioat,
O,O-Diethyl-O-(2-pyrazinyl)-phosphorthioat,
O,O-Diethyl-O-[2-isopropyl-4-methyl-pyrimidinyl-(6)]-phosphorthioat,
O,O-Diethyl-O-[2-(diethylamino)-6-methyl-4-pyrimidinyl]-thionophosphat,
O,O-Dimethyl-S-(4-oxo-1,2,3-benzotriazin-3-[4H]-yl-methyl)-phosphordithioat,
O,O-Dimethyl-S-[(4,6-diamino-1,3,5-triazin-2-yl)-methyl]-phosphordithioat,
O,O-Diethyl-(1-phenyl-1,2,4-triazol-3-yl)-thionophosphat,
O,S-Dimethyl-phosphor-amido-thioat,
O,S-Dimethyl-N-acetyl-phosphoramidothioat,
γ-Hexachlorcyclohexan,
1,1-Di-(p-methoxyphenyl)-2,2,2-trichlor-ethan,
6,7,8,9,10,10-Hexachloro-1,5,5a,6,9,9a-hexahydro-6,9-methano-2,4,3-benzodioxa-thiepin-3-oxid,
Pyrethrine,
DL-2-Allyl-3-methyl-cyclopenten-(2)-on-(1)-yl-(4)-DL-cis,trans-chrysanthemat,
5-Benzyl-furyl-(3)-methyl-DL-cis,trans-chrysanthemat,
3-Phenoxybenzyl(±)-cis,trans-2,2-dimethyl-3-(2,2-dichlorvinyl)-cyclopropancarboxylat,
-Cyano-3-phenoxybenzyl(±)-cis,trans-2,2-dimethyl-3-(2,2-dichlorvinyl)-cyclopropancarboxylat,
(s)- -Cyano-3-phenoxybenzyl-cis(1R,3R)-2,2-dimethyl-3-(2,2-dibromvinyl)-cyclopropancarboxylat,
3,4,5,6-Tetrahydrophthalimidoethyl-DL-cis,trans-chrysanthemat,
2-Methyl-5-(2-propinyl)-3-furylmethyl-chrysanthemat,
(α-Cyano-3-phenoxybenzyl)-α-isopropyl-4-chlorphenylacetat.

Die folgenden Beispiele belegen die biologische Wirkung der neuen Verbindungen. Die Numerierung der Wirkstoffe entspricht der der tabellarischen Auflistung.

## Beispiel 1

### Zuchtversuch mit Moskito-Larven (Aedes aegypti)

200 ml Leitungswasser werden mit der Wirkstoffaufbereitung, die 10 Gew.-% Wirkstoff und 90 Gew.-% eines Emulgatorgemisches aus 10 Gew.-% ethoxyliertem Rizinusöl, 20 Gew.-% ethoxyliertem Isooctylphenol und 70 Gew.-% Cyclohexanon enthält, versetzt und darauf mit 30 bis 40 Aedes-Larven im 4-Stadium belegt.

Die Versuchstemperatur beträgt 25°C. Beurteilt werden Verpuppung und Schlüpfen der Imagines, wobei eine unbehandelte Kontrolle als Maßstab dient. Während der Versuchsdauer von 10 bis 12 Tagen wird einmal mit einem herkömmlichen pulverisierten Fischfutter gefüttert.

In diesem Test zeigen die Wirkstoffe Nr. 1, 2, 3, 6, 8, 9, 15, 24, 28, 32 eine bessere Wirkung als bekannte substituierte N-Benzoyl-N'-phenyl-harnstoffe.

## Beispiel 2

### Zuchtversuch mit Maiszünsler-Larven (Ostrinia nubilalis)

Die Zucht erfolgt auf einem Nährboden folgender Zusammensetzung:

515 g Maismehl
130 g Weizenkeime
137 g Bierhefe
18 g Ascorbinsäure
10 g Cellulosepulver
5 g p-Hydroxibenzoesäuremethylester
20 g Spurenelemente nach Wesson
20 ml Vitaminlösung
80 g Agar
3100 ml Wasser

Je 50 ml des Nährbodens füllt man in 100 ml Plastikbecher und mischt die wäßrige Wirkstoffaufbereitung sorgfältig unter.

Nach dem Erkalten belegt man jedes Gefäß mit 4 Raupen (L 3). Pro Konzentration werden 5 Gefäße angesetzt. Die Beobachtung erstreckt sich bis zum Schlüpfen der Falter.

In diesem Test zeigen die Wirkstoffe Nr. 1, 6, 15, 18 und 22 eine bessere Wirkung als bekannte substituierte N-Benzoyl-N'-phenyl-harnstoffe.

## Patentansprüche

1. N-Benzoyl-N'-phenoxyphenylharnstoffe der Formel

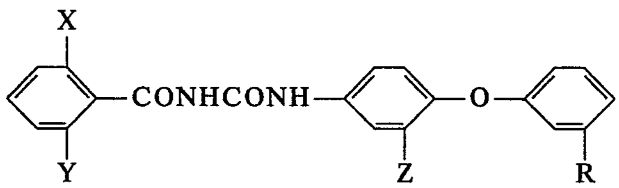

(I)

in der

X für Chlor, Fluor oder Methyl,
Y für Wasserstoff, Fluor,
Z für Wasserstoff, Fluor, Chlor, Brom, Alkyl mit 1 bis 8 Kohlenstoffatomen oder Trifluormethyl, und
R für Halogen, Alkyl mit 1 bis 8 Kohlenstoffatomen, Alkoxy, Halogenalkyl, Halogenalkoxy, Alkylmercapto oder Halogenalkylmercapto mit 1 bis 4 Kohlenstoffatomen stehen.

2. N-Benzoyl-N'-phenoxyphenylharnstoffe der Formel I gemäß Anspruch 1, dadurch gekennzeichnet, daß

X für Chlor oder Fluor,
Y für Wasserstoff oder Fluor,
Z für Wasserstoff, Fluor oder Chlor, und
R für Methyl, Fluor, Chlor oder Trifluormethyl stehen.

3. Insektizides Mittel, enthaltend einen N-Benzoyl-N'-phenoxyphenylharnstoff der Formel I gemäß Anspruch 1.

4. Insektizides Mittel, enthaltend inerte Zusatzstoffe und einen N-Benzoyl-N'-phenoxyphenylharnstoff der Formel I gemäß Anspruch 1.

5. Verfahren zur Bekämpfung von Insekten, dadurch gekennzeichnet, daß man eine insektizid wirksame Menge eines N-Benzoyl-N-phenoxyphenylharnstoffs der Formel I gemäß Anspruch 1 auf die Insekten und/oder deren Lebensraum einwirken läßt.

6. Verfahren zur Herstellung eines insektiziden Mttels, dadurch gekennzeichnet, daß man mindestens einen N-Benzoyl-N'-phenoxyphenylharnstoff der Formel I gemäß Anspruch 1 mit oberflächenaktiven Verbindungen und/oder Streckmitteln und/oder Synergisten mischt.

7. Verfahren zur Herstellung von N-Benzoyl-N'-phenoxyphenylharnstoffen der Formel I gemäß Anspruch 1, dadurch gekennzeichnet, daß man ein Benzoylisocyanat der Formel

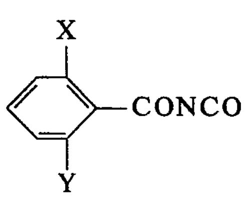

(II)

in der X und Y die im Anspruch 1 genannten Bedeutungen haben, mit einem phenoxysubstituierten Anilin der Formel

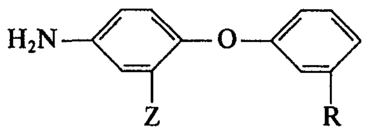

(III)

in der Z und R die im Anspruch 1 genannten Bedeutungen haben, in Gegenwart eines inerten organischen Lösungsmittels bei einer Temperatur im Bereich zwischen 0 und 80°C umsetzt.

8. Verfahren zur Herstellung von N-Benzoyl-N'-phenoxyphenylharnstoffen der Formel I gemäß Anspruch 1, dadurch gekennzeichnet, daß man ein Amid der Formel

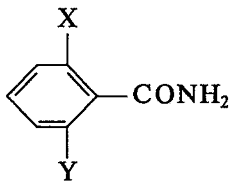

(IV)

in der X und Y die im Anspruch 1 genannten Bedeutungen haben, mit einem Isocyanat der Formel

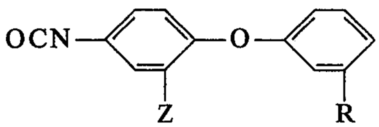

(V)

in der Z und R die im Anspruch 1 genannte Bedeutung haben, gegebenenfalls in Gegenwart eines inerten organischen Lösungsmittels bei einer Temperatur zwischen 0 und 140°C umsetzt.

**Claims**

1. N-Benzoyl-N'-phenoxyphenylureas of the formula

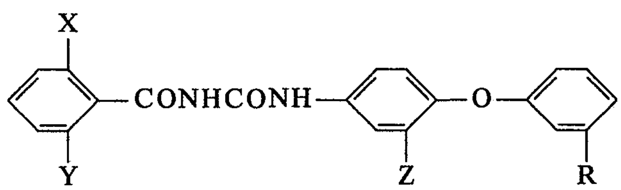

(I)

where X is chlorine, fluorine or methyl, Y is hydrogen or fluorine, Z is hydrogen, fluorine, chlorine, bromine, alkyl of 1 to 8 carbon atoms or trifluoromethyl, and R is halogen, alkyl of 1 to 8 carbon atoms, or alkoxy, haloalkyl, haloalkoxy, alkylmercapto or haloalkylmercapto, each of 1 to 4 carbon atoms.

2. N-Benzoyl-N'-phenoxyphenylureas of the formula I as claimed in claim 1, wherein X is chlorine or fluorine, Y is hydrogen or fluorine, Z is hydrogen, fluorine or chlorine, and R is methyl, fluorine, chlorine or trifluoromethyl.

3. An insecticidal agent containing an N-benzoyl-N'-phenoxy-phenylurea of the formula I as claimed in claim 1.

4. An insecticidal agent containing inert additives and an N-benzoyl-N'-phenoxyphenylurea of the formula I as claimed in claim 1.

5. A process for combating insects, wherein an insecticidally effective amount of an N-benzoyl-N'-phenoxyphenylurea of the formula I as claimed in claim 1 is allowed to act on the insects and/or their habitat.

6. A process for producing an insecticidal agent, wherein at least one N-benzoyl-N'-phenoxyphenylurea of the formula I as claimed in claim 1 is mixed with surfactants and/or extenders and/or synergists.

7. A process for the production of N-benzoyl-N'-phenoxyphenylureas of the formula I as claimed in claim 1, wherein a benzoyl isocyanate of the formula

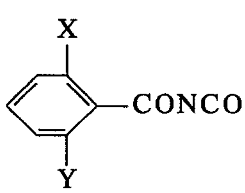

(II)

where X and Y have the meanings given in claim 1, is reacted with a phenoxy-substituted aniline of the formula

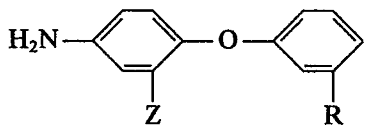 (III)

where Z and R have the meanings given in claim 1, in the presence of an inert organic solvent at from 0 to 80°C.

8. A process for the production of N-benzoyl-N'-phenoxyphenylureas of the formula I as claimed in claim 1, wherein an amide of the formula

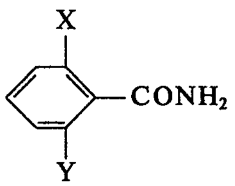 (IV)

where X and Y have the meanings given in claim 1, is reacted with an isocyanate of the formula

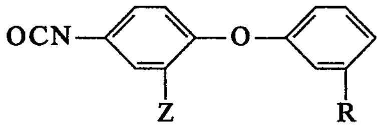 (V)

where Z and R have the meanings given in claim 1, in the presence or absence of an inert organic solvent at from 0 to 140°C.

**Revendications**

1. N-Benzoyl-N'-phénoxyphényl-urées de la formula

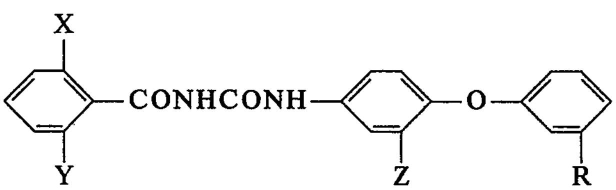 (I)

dans laquelle

X chlore, fluor ou méthyle,
Y hydrogène ou fluor,
Z hydrogène, fluor, chlore, brome, alkyle en $C_1$ à $C_8$ ou trifluorométhyle et
R halogène, alkyle en $C_1$ à $C_8$, alcoxy, halo-alkyle, halo-alcoxy, alkyl-mercapto ou halo-alkyl-mercapto en $C_1$ à $C_4$.

2. N-Benzoyl-N'-phénoxyphényl-urées de la formule I selon la revendication 1, caractérisées en ce que

X chlore ou fluor,
Y hydrogène ou fluor,
Z hydrogène, fluor ou chlore et
R méthyle, fluor, chlore ou trifluorométhyle.

3. Composition insecticide, contenant une N-benzoyl-N'-phénoxyphényl-urée de la formule I selon la revendication 1.

4. Composition insecticide, contenant une N-benzoyl-N'-phénoxyphényl-urée de la formule I selon la revendication 1 et des additifs inertes.

5. Procédé de lutte contre des insectes, caractérisé en ce que l'on fait agir sur les insectes et(ou) leur biotope une quantité efficace du point de vue insecticide d'une N-benzoyl-N'-phénoxyphényl-urée de la formule I selon la revendication 1.

6. Procédé de préparation d'une composition insecticide, caractérisé en ce que l'on mélange au moins une N-benzoyl-N'-phénoxyphényl-urée de la formule I selon la revendication 1 à des agents tensio-actifs et(ou) des matières de charge et(ou) des agents de potentialisation.

7. Procédé de préparation de N-benzoyl-N'-phénoxyphényl-urées de la formule I selon la revendication 1, caractérisé en ce que l'on fait réagir un isocyanate de benzoyle de la formule

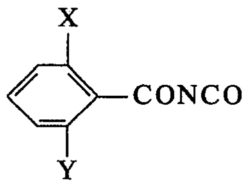

(II)

dans laquelle X et Y possèdent les significations définies dans la revendication 1, dans un solvant organique inerte à une température comprise dans la gamme de 0 à 80°C, avec une aniline phénoxy-substituée de la formule

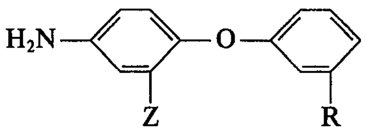

(III)

dans laquelle Z et R possèdent les significations définies dans la revendication 1.

8. Procédé de préparation de N-benzoyl-N'-phénoxyphényl-urées de la formule I selon la revendication 1, caractérisé en ce que l'on fait réagir un amide de la formule

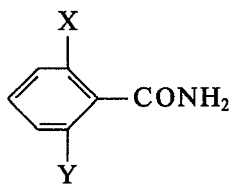

(IV)

dans laquelle X et Y possèdent les significations définies, à une température comprise entre 0 et 140°C, éventuellement dans un solvant organique inerte, avec un isocyanate de la formule

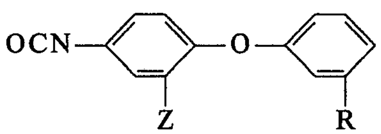

(V)

dans laquelle Z et R possèdent la signification définie dans la revendication 1.